# FASCICULE DE BREVET EUROPEEN

(11) **EP 3 123 544 B1**
(45) Date de publication et mention de la délivrance du brevet: **19.06.2019**
(21) Numéro de dépôt: 15718517.4
(22) Date de dépôt: 23.03.2015
(51) Int. Cl.: H01M 4/86, H01M 4/88, H01M 4/90, H01M 8/16

(54) **REACTEUR IMPLANTABLE BIOCOMPATIBLE**
IMPLANTIERBARER BIOKOMPATIBLER REAKTOR
IMPLANTABLE BIOCOMPATIBLE REACTOR

(30) Priorité: 25.03.2014 FR 1452534
(43) Date de publication de la demande: 01.02.2017
(73) Titulaire: Université Grenoble Alpes, 38400 Saint Martin d'Hères (FR)
(72) Inventeur: EL ICHI, Sarra, 38706 La Tronche Cedex (FR); MARTIN, Donald K., 38706 La Tronche Cedex (FR); CINQUIN, Philippe, 38706 La Tronche Cedex (FR); ZEBDA, Abdelkader, 38706 La Tronche Cedex (FR)
(74) Mandataire: Cabinet Chaillot
(86) Numéro de dépôt international: PCT/FR2015/050725
(87) Numéro de publication internationale: WO 2015/145054

(56) Documents cités:
- WO-A2-2013/130145
- FR-A1- 2 958 801
- FR-A1- 2 963 989
- US-A- 5 885 609
- US-A1- 2011 039 164
- HOLZINGER MICHAEL ET AL: "Carbon nanotube/enzyme biofuel cells", ELECTROCHIMICA ACTA, vol. 82, 3 mars 2012 (2012-03-03), pages 179-190, XP028939099, ISSN: 0013-4686, DOI: 10.1016/J.ELECTACTA.2011.12.135

## Description

La présente demande de brevet revendique la priorité de la demande de brevet français FR14/52534 qui sera considérée comme faisant partie intégrante de la présente description.

### Domaine

La présente invention concerne un réacteur implantable in vivo, au niveau duquel est susceptible de se produire une réaction entre des éléments confinés dans ce réacteur et des composés présents dans l'organisme hôte. Cette réaction peut par exemple conduire à une déformation du réacteur, à la génération d'un potentiel électrique, ou à la transformation chimique du composé interagissant avec le réacteur.

Un réacteur conduisant à la génération d'un potentiel électrique pourra constituer une électrode d'une biopile ou d'un biocapteur, de type à sucre-oxygène, par exemple à glucose-oxygène.

Un réacteur conduisant à la transformation chimique d'un composé interagissant avec le réacteur constituera par exemple un destructeur de glucose (glucose killer) en transformant par exemple du glucose en un composé qui sera éliminé par l'organisme.

Bien que l'invention et l'état de la technique soient décrits ici principalement dans le cas de bioélectrodes d'une biopile, on comprendra que l'invention s'applique de façon générale à tout réacteur implantable in vivo.

### Exposé de l'art antérieur

Divers types de biopiles à glucose-oxygène sont décrits dans l'art antérieur, par exemple dans la demande de brevet PCT/FR2009/050639 (B8606). Dans ces biopiles connues, chaque électrode, anode et cathode, correspond à une enceinte contenant un milieu liquide dans lequel plonge un fil d'électrode. Les enceintes d'anode et de cathode sont délimitées par des membranes pouvant être traversées par l'hydrogène et l'oxygène mais évitant la circulation d'autres éléments plus lourds.

L'anode comprend dans une solution une enzyme et un médiateur redox. L'enzyme est apte à catalyser l'oxydation du sucre et est par exemple choisie dans le groupe comprenant glucose-oxydase si le sucre est du glucose et lactose-oxydase si le sucre est du lactose. Le médiateur redox a un potentiel redox bas susceptible d'échanger des électrons avec l'enzyme d'anode et est par exemple choisi dans le groupe comprenant : ubiquinone (UQ) et ferrocène.

La cathode comprend également dans une solution une enzyme et de préférence un médiateur redox. L'enzyme est apte à catalyser la réduction de l'oxygène et est par exemple choisie dans le groupe comprenant : polyphénol oxydase (PPO), laccase et bilirubine oxydase. Le médiateur redox a un potentiel redox haut susceptible d'échanger des électrons avec l'enzyme de cathode et est par exemple choisi dans le groupe comprenant : hydroquinone (QH₂) et 2,2'-azinobis-(3-éthylbenzo-thiazoline-6-sulfonate) (ABTS) .

Il se produit alors au niveau de l'anode et de la cathode des réactions du type suivant :
Cathode : 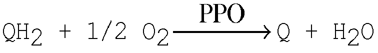
Anode : 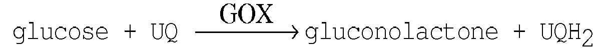
Cathode :

   Q + 2H⁺ + 2e⁻→QH₂
Anode :

   UQH₂→UQ + 2H⁺ + 2e⁻
ces réactions étant données dans le cas particulier où le sucre est du glucose, l'enzyme d'anode est de la glucose-oxydase (GOX), le médiateur redox d'anode est de l'ubiquinone (UQ), l'enzyme de cathode est de la polyphénol oxydase (PPO), et le médiateur redox de cathode est de la quinhydrone (QH₂). On obtient alors un potentiel d'anode de 20 mV et un potentiel de cathode de 250 mV, ce qui conduit à une différence de potentiel à courant nul de la biopile de 230 mV.

De telles biopiles fonctionnent convenablement mais, notamment en ce qui concerne la biopile décrite dans la demande de brevet PCT/FR2009/050639, nécessitent que des conducteurs d'anode et de cathode trempent dans des enceintes contenant des liquides appropriés, ce qui constitue un inconvénient pratique dans de nombreux cas et rend notamment très difficile sinon impossible d'implanter de telles biopiles dans un être vivant.

En effet, on cherche à implanter de telles biopiles dans des êtres vivants, notamment pour alimenter divers actionneurs, tels que des stimulateurs cardiaques, des sphincters artificiels, ou même des coeurs artificiels.

On a proposé des biopiles à électrodes solides. Toutefois, des biopiles utilisant de telles électrodes, notamment quand elles sont implantées dans un être vivant, ont présenté une faible durée de vie.

Une biopile à glucose-oxygène implantable in vivo est décrite notamment dans le brevet européen EP 2 375 481 de la demanderesse (B10272). Le contenu de ce brevet sera considéré ici comme connu et comme faisant partie intégrante de la présente description.

Dans ce brevet, il est proposé de fabriquer des pastilles d'anode et de cathode d'une biopile à partir d'une compression d'un conducteur tel que du graphite et d'une enzyme auxquels est ajouté un médiateur redox. La cathode et l'anode, ainsi que de préférence l'ensemble de l'anode et de la cathode, sont entourés d'une enceinte semi-perméable, par exemple du type utilisé en dialyse, pour laisser passer le glucose et l'oxygène et ne pas laisser passer les enzymes et les médiateurs redox. Le matériau conducteur à partir duquel sont effectuées la compression d'anode et la compression de cathode est indiqué comme étant du graphite ou un polymère conducteur.

La figure 1 ci-jointe reproduit la figure 2 de ce brevet antérieur. On y voit une pastille d'anode A et une pastille de cathode K solidaires respectivement de conducteurs 1 et 3. L'anode est entourée d'une membrane semi-perméable 11, la cathode d'une membrane semi-perméable 12 et l'ensemble est entouré d'une membrane semi-perméable 13.

Des résultats expérimentaux in vivo satisfaisants ont été obtenus avec les électrodes de biopile décrites dans ce brevet.

Il est toutefois souhaitable d'améliorer encore la durée de vie des électrodes, c'est-à-dire la durée de fonctionnement de la biopile et d'améliorer au maximum la biocompatibilité de cette pile.

Plus généralement, il est souhaitable d'améliorer la durée de vie de bioréacteurs tels que définis ci-dessus.

Les documents US2011/039164, FR2963989, WO2013/130145 et l'article de Holzinger Michael et al. intitulé : « Carbon nanotube/enzyme biofuel cells » Electrochimica acta, vol. 82, 3 mars 2012 concernent diverses réalisations de bioréacteurs.

### Résumé

Ainsi, on prévoit un procédé de fabrication d'un bioréacteur comprenant les étapes suivantes :
préparer un mélange de poudres dans laquelle la proportion de poudre d'enzyme par rapport à une poudre de nanotubes de carbone est de l'ordre de 50/50 en poids ;
préparer une solution visqueuse de chitosane dans un rapport de 5 à 15 (en mg) de chitosane à 0,75 à 1,25 (en ml) d'acide acétique dilué à 0,4 à 0,6 % en volume dans de l'eau ;
ajouter au mélange de poudres la chitosane visqueuse dans une proportion pondérale de 3 à 5 pour la poudre à 5 à 10 pour la chitosane ;
procéder à une première compression puis à un broyage léger ;
procéder à une deuxième compression pour réaliser une pastille, la pression appliquée pendant la première et la deuxième compressions étant située dans une plage de 2000 à 6000 kPa; et
sécher à température ambiante.

Selon un mode de réalisation, la solution comprend de 0,002 à 0,005 % en masse par volume de génipine.

Selon un mode de réalisation, la solution comprend de 0,001 à 0,005 % en masse par volume d'acide caféique.

On prévoit aussi un bioréacteur obtenu par la mise en oeuvre du procédé selon la présente invention.

On prévoit aussi un bioréacteur dans lequel une membrane poreuse à base de chitosane est posée sur une face active du bioréacteur et collée à la périphérie de celle-ci.

Selon un mode de réalisation, le bioréacteur constitue une bioélectrode en forme de pastille, dans laquelle un conducteur est collé par l'intermédiaire d'une colle conductrice à la face de la pastille opposée à la face active.

Selon un mode de réalisation, la membrane comprend des pores d'un diamètre moyen de l'ordre de 1 à 10 nanomètres.

Selon un mode de réalisation, la membrane comprend une face lisse tournée vers la pastille et une face rugueuse tournée vers l'extérieur.

On prévoit aussi un bioréacteur selon l'invention, dans lequel la membrane poreuse est obtenue par un procédé comprenant les étapes suivantes :
préparer une solution dans un rapport de 5 à 15, (en mg) de chitosane à 0,75 à 1,25 (en ml) d'acide acétique dilué à 0,4 à 0,6 % dans de l'eau ;
agiter ;
verser sur un support lisse ; et
sécher pendant une durée de 2 à 4 jours à température ambiante.

### Brève description des dessins

Ces caractéristiques et avantages, ainsi que d'autres, seront exposés en détail dans la description suivante de modes de réalisation particuliers faite à titre non limitatif en relation avec les figures jointes parmi lesquelles :
la figure 1 correspond à la figure 2 du brevet européen EP 2 375 481 ;
les figures 2A et 2B sont respectivement une vue en coupe et une vue de dessus d'un mode de réalisation d'une électrode ; et
la figure 3 représente des caractéristiques de courant en fonction du temps de diverses biopiles.

### Description détaillée

Tout d'abord, on prévoit ici de fabriquer une pastille de bioélectrode non pas à partir de la compression de seulement un conducteur et une enzyme, mais à partir d'une compression de chitosane, d'une enzyme et d'un conducteur et éventuellement d'un médiateur redox et d'autres additifs. Le conducteur peut avantageusement être constitué de nanotubes de carbone multiparois (MWCNT - MultiWalled Carbon NanoTubes).

On prépare initialement un mélange de poudres d'une enzyme et de nanotubes de carbone, la proportion de poudre d'enzyme par rapport à la poudre de nanotubes de carbone étant de l'ordre de 50/50 en poids, cette proportion pouvant varier d'environ 20 %.

On prépare également une solution visqueuse de chitosane en ajoutant de la poudre de chitosane dans de l'acide acétique dilué à 0,5 % en volume chauffé à 50°C, et en agitant pendant 2 heures à température ambiante.

On ajoute au mélange de poudres la chitosane visqueuse dans une proportion pondérale de 2 pour la poudre à 3 pour la chitosane. Pour une pastille on utilisera par exemple 0,04 gramme de poudre et 0,06 gramme de chitosane.

On réalise un mélange de la poudre et de la chitosane à l'état visqueux et on procède à une première compression puis à un broyage léger. On réalise ensuite une deuxième compression, qui, comme la première compression, est effectuée à une pression choisie dans une plage de 2000 à 6000 kPa pour fournir une pastille, puis on procède à un séchage pendant deux à quatre jours à température ambiante (20 à 30°C) pour que l'ensemble se polymérise.

On pourra ajouter au mélange initial un agent de réticulation, par exemple de la génipine à 0,0045 % en masse par volume (g/100 ml) dans la solution visqueuse de chitosane après 2h d'agitation. La génipine est préalablement solubilisée dans une solution de 12 % de diméthylsulfoxyde (DMSO) et 88 % d'eau (H₂O). On pourra également pour améliorer la résistance de la membrane aux acides ajouter au mélange initial un produit tel que de l'acide caféique dans une proportion de 0,0032 % en masse par volume (g/100 ml) dans la solution visqueuse de chitosane. L'acide caféique est préalablement solubilisé à 4 % dans de l'éthanol. On laisse agiter la solution 30 min avant de prélever 3 g à étaler sur le support lisse pour le séchage comme décrit précédemment.

Une caractéristique du procédé décrit ici est que, lors de la deuxième compression et du séchage, la chitosane se met en fibres longues interconnectées d'un diamètre d'environ 30 nm. On soulignera le fait qu'un réseau tridimensionnel nanofibreux et nanoporeux est obtenu simplement par compression du polymère avec la poudre et évaporation du solvant à température ambiante. Il en résulte que l'enzyme et les nanotubes de carbone sont immobilisés dans la matrice de fibres de chitosane et ne migrent pas vers l'extérieur de la pastille. Ceci présente un avantage important car l'enzyme, piégée par la matrice de fibres, reste protégée et active pendant une longue durée. De plus, il convient de piéger les nanotubes de carbone, des questions se posant actuellement sur la nocivité éventuelle des nanotubes de carbone in vivo.

Une biocathode comprenant seulement un conducteur et une enzyme, telle que décrite dans le brevet antérieur susmentionné, présente une stabilité de 1 mois en fonctionnement discontinu. Une biocathode à base de chitosane-MWCNT-laccase, telle que décrite ici, présente une stabilité supérieure à 2 mois en fonctionnement continu. Des mesures in vitro montrent que la stabilité en fonctionnement discontinu de la biocathode décrite ici dépasse les six mois. De plus cette stabilité est aussi assurée in vivo pour une période supérieure à 200 jours. Ceci démontre que la bioélectrode décrite ici offre à l'enzyme un environnement protecteur à son activité mais aussi retient l'enzyme à l'intérieur de la pastille d'électrode et à proximité des nanotubes de carbone pour la conduction électrique. La porosité de la matrice tridimensionnelle de chitosane permet une bonne diffusion des substrats de l'enzyme.

Le procédé de fabrication des électrodes à base de polymère conducteur et d'enzyme peut être appliqué à la cathode ou à l'anode. Différentes enzymes peuvent être immobilisées dans cette structure : laccase, bilirubine-oxydase, polyphénol-oxydase, glucose-oxydase, glucose-déshydrogénase, catalase, peroxydase.

Selon un autre aspect de la présente invention, on prévoit une membrane filtrante particulière pour une bioélectrode enzymatique telle que ci-dessus ou toute autre bioélectrode enzymatique obtenue par compression d'un conducteur, d'une enzyme et éventuellement d'un médiateur redox - le médiateur redox n'étant pas indispensable dans la cathode.

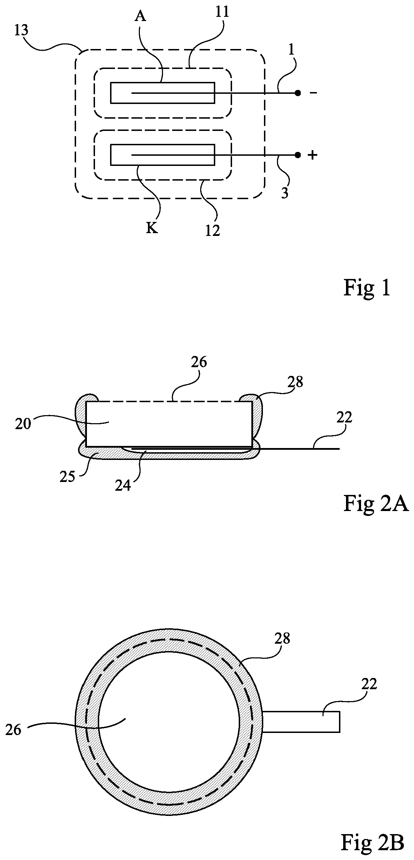

Comme l'illustrent les figures 2A et 2B, l'électrode se présente sous forme d'une pastille 20 ayant par exemple une forme circulaire en vue de dessus, un diamètre de 0,5 à 1 cm et une épaisseur de 0,5 à 2 mm. Sur la face inférieure de la pastille 20 est fixé un ruban conducteur 22 par exemple par une colle conductrice, par exemple une pâte de carbone 24 elle-même revêtue d'une couche protectrice de colle au silicone 25, choisie parmi des colles biocompatibles. Sur la face supérieure de la pastille 20 est posée - et non pas déposée - une membrane 26 collée à sa périphérie à la pastille par un anneau de colle au silicone 28.

On prévoit ici d'utiliser pour la membrane 26 une membrane à base de chitosane. Cette membrane est par exemple obtenue en partant d'une solution dans un rapport de 5 à 15, par exemple 10 (en mg) de chitosane à 0,75 à 1,25, par exemple 1 (en ml) d'acide acétique dilué à 0,4 à 0,6 % en volume dans de l'eau et chauffé à 50°C. Dans un essai, on a réalisé une dissolution de 200 mg de chitosane dans 20 ml d'acide acétique dilué à 0,5 % en volume dans de l'eau. Ce mélange est agité pendant deux heures. Ensuite, on a prélevé 3 g de ce mélange qu'on a étalé sur un support lisse non adhésif (diamètre 28 cm), par exemple une coupelle de polystyrène antistatique, et séché pendant une durée de 2 à 4 jours à température ambiante, par exemple à une température de 20 à 30°C. Dans un essai, on a séché pendant trois jours à 25°C.

On obtient ainsi un film nanoporeux flexible. Des expériences effectuées par la demanderesse ont montré que cette flexibilité était liée au fait que le séchage est effectué pendant une longue durée à température ambiante. Cette caractéristique n'est pas obtenue par exemple si l'on utilise des températures de séchage supérieures à 40°C. Pour une épaisseur de film de l'ordre de 7 à 15 pm, par exemple 10 pm, on a obtenu une membrane poreuse avec des diamètres moyens de pores de l'ordre de 1 à 10 nanomètres. On préfèrera se placer dans des conditions où ce diamètre moyen est de l'ordre de 5 à 8 nm pour permettre de laisser passer le glucose et de filtrer les composés de plus grandes dimensions.

Comme dans le cadre de la réalisation d'une pastille, on pourra ajouter au mélange initial un agent de réticulation, par exemple de la génipine, et un agent de résistance aux acides, par exemple de l'acide caféique.

Le film obtenu présente une différence de rugosité entre les deux faces qui est due au fait que l'une des faces (la plus rugueuse) a été au contact de l'air et non du support (face lisse). Lors du montage, on placera de préférence la face rugueuse vers l'extérieur par rapport à la surface de la pastille de bioélectrode. En effet, la différence de rugosité sur un film mince influence la diffusion ionique et par conséquent, la résistivité électrique. Les inventeurs ont montré que le film de chitosane présente une bonne conductivité ionique (10⁻⁴ S.cm⁻¹). Cette conductivité ionique est meilleure que celle obtenue avec des membranes commerciales telles que le Nafion ou l'acétate de cellulose.

Le film de chitosane décrit ici permet, par ses propriétés mécaniques notamment sa flexibilité et son taux de gonflement adéquat, de conférer une stabilité mécanique à l'électrode en épousant la surface de la pastille après gonflement de celle-ci dans le liquide. Il confère une interface biocompatible au contact des tissus après implantation de la biopile. Il constitue une barrière efficace contre un éventuel relargage des constituants de l'électrode d'une part, et contre les molécules biologiques venant du liquide extra-cellulaire.

On pourra régler le diamètre des pores en modifiant : la concentration de la chitosane dans le solvant (acide acétique), le ratio chitosane/agent de réticulation, le poids moléculaire de la chitosane en poudre mis dans la solution initiale d'acide acétique.

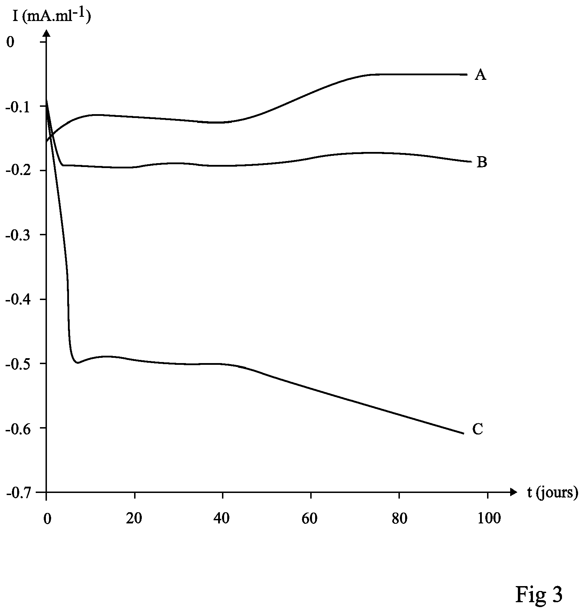

La figure 3 représente une caractéristique de courant en mA/ml (les millilitres correspondant au volume de la pastille) en fonction du temps en jours pour diverses biopiles. La courbe A correspond au cas où on a utilisé une membrane d'acétate de cellulose, la courbe B au cas où on a utilisé une membrane de Nafion, et la courbe C au cas où on a utilisé une membrane à base de chitosane telle que décrite précédemment. On constate que le courant (négatif) est nettement plus important dans le cas de la membrane à base de chitosane et que les caractéristiques de la biocellule ne se dégradent pas, bien au contraire, en fonction du temps. Par contre, on note que pour l'acétate de cellulose, la caractéristique part d'une valeur de -0,15 et chute à une valeur de l'ordre de -0,05 au bout d'environ 70 jours. Avec une membrane en Nafion, on obtient une caractéristique relativement constante mais des densités de courant deux à trois fois plus faibles qu'avec une membrane à base de chitosane.

Bien que l'invention et l'état de la technique soient décrits ici principalement dans le cas d'une bioélectrode, on comprendra que l'invention s'applique de façon générale à tout bioréacteur implantable in vivo, tel que défini en tête de la présente description.

## Revendications

1. Procédé de fabrication d'un bioréacteur comprenant les étapes suivantes :
préparer un mélange de poudres dans laquelle la proportion d'une poudre d'enzyme par rapport à une poudre de nanotubes de carbone est de l'ordre de 50/50 en poids ;
préparer une solution visqueuse de chitosane dans un rapport de 5 à 15 (en mg) de chitosane à 0,75 à 1,25 (en ml) d'acide acétique dilué à 0,4 à 0,6 % en volume dans de l'eau ;
ajouter au mélange de poudres la chitosane visqueuse dans une proportion pondérale de 3 à 5 pour la poudre à 5 à 10 pour la chitosane ;
procéder à une première compression puis à un broyage léger ;
procéder à une deuxième compression pour réaliser une pastille, la pression appliquée pendant la première et la deuxième compression étant située dans une plage de 2000 à 6000 kPa ; et
sécher à température ambiante.

2. Procédé selon la revendication 1, dans lequel la solution comprend de 0,002 à 0,005 % en masse par volume de génipine.

3. Procédé selon la revendication 1, dans lequel la solution comprend de 0,001 à 0,005 % en masse par volume d'acide caféique.

4. Bioréacteur obtenu par la mise en oeuvre du procédé selon l'une quelconque des revendications 1 à 3.

5. Bioréacteur selon la revendication 4, dans lequel une membrane poreuse (26) à base de chitosane est posée sur une face active du bioréacteur et collée à la périphérie de celle-ci.

6. Bioréacteur selon la revendication 5 constituant une bioélectrode en forme de pastille (20), dans laquelle un conducteur (22) est collé par l'intermédiaire d'une colle conductrice (24) à la face de la pastille opposée à la face active.

7. Bioréacteur selon la revendication 5 ou 6, dans lequel la membrane comprend des pores d'un diamètre moyen de l'ordre de 1 à 10 nanomètres.

8. Bioréacteur selon l'une quelconque des revendications 5 à 7, dans lequel la membrane comprend une face lisse tournée vers la pastille et une face rugueuse tournée vers l'extérieur.

9. Bioréacteur selon l'une quelconque des revendications 5 à 8, dans lequel la membrane poreuse est obtenue par un procédé comprenant les étapes suivantes :
préparer une solution dans un rapport de 5 à 15, (en mg) de chitosane à 0,75 à 1,25 (en ml) d'acide acétique dilué à 0,4 à 0,6 % dans de l'eau ;
agiter ;
verser sur un support lisse ; et
sécher pendant une durée de 2 à 4 jours à température ambiante.

## Patentansprüche

1. Verfahren zur Herstellung eines Bioreaktors, umfassend die Folgenden Schritte:
Zubereiten einer Mischung aus Pulvern, wobei die Proportion eines Enzympulvers mit Bezug auf ein Kohlenstoffnanoröhrchen-Pulver im Bereich von 50/50, bezogen auf das Gewicht, ist;
Zubereiten einer viskosen Chitosanlösung in einem Verhältnis von 5 bis 15 (in mg) Chitosan zu 0,75 bis 1,25 (in ml) Essigsäure, verdünnt zu 0,4 bis 0,6 Vol.% in Wasser;
Zugeben zur Mischung aus Pulvern das viskose Chitosan in einer Gewichtsproportion von 3 bis 5 für das Pulver zu 5 bis 10 für das Chitosan;
Durchführen einer ersten Kompression, dann einer leichten Zerkleinerung;
Durchführen einer zweiten Kompression, um eine Pastille zu erzeugen, wobei der angewendete Druck während der ersten und der zweiten Kompression in einem Bereich von 2000 bis 6000 kPa liegt; und
Trocknen bei Umgebungstemperatur.

2. Verfahren nach Anspruch 1, wobei die Lösung 0,002 bis 0,005 Massenprozent pro Volumen Genipin umfasst.

3. Verfahren nach Anspruch 1, wobei die Lösung 0,001 bis 0,005 Massenprozent pro Volumen Kaffeesäure umfasst.

4. Bioreaktor, erhalten durch das Ausführen des Verfahrens nach einem der Ansprüche 1 bis 3.

5. Bioreaktor nach Anspruch 4, wobei eine poröse Membran (26) auf der Grundlage von Chitosan auf eine aktive Seite des Bioreaktors gestellt und an den Umfang dieses geklebt wird.

6. Bioreaktor nach Anspruch 5, der eine Bioelektrode in Form einer Pastille (20) darstellt, in der ein Leiter (22) mit Hilfe eines leitenden Klebers (24) an die Seite der Pastille gegenüber der aktiven Seite geklebt wird.

7. Bioreaktor nach Anspruch 5 oder 6, wobei die Membran Poren mit einem mittleren Durchmesser im Bereich von 1 bis 10 Nanometern umfasst.

8. Bioreaktor nach einem der Ansprüche 5 bis 7, wobei die Membran eine glatte Seite umfasst, die gegen die Pastille gedreht ist, und eine raue Seite, die gegen die Außenseite gedreht ist.

9. Bioreaktor nach einem der Ansprüche 5 bis 8, wobei die poröse Membran durch ein Verfahren erhalten wird, das die folgenden Schritte umfasst:
Zubereiten einer Lösung in einem Verhältnis von 5 bis 15 (in mg) Chitosan zu 0,75 bis 1,25 (in ml) Essigsäure, verdünnt zu 0,4 bis 0,6 % in Wasser;
Schütteln;
Gießen auf einen glatten Träger; und
Trocknen während einer Dauer von 2 bis 4 Tagen bei Raumtemperatur.

## Claims

1. A method of manufacturing a bioreactor comprising the steps of:
preparing a mixture of powders where the proportion of an enzyme powder relative to a carbon nanotube powder is of the order of 50/50 by weight;
preparing a viscous solution of chitosan in a ratio of 5 to 15 (in mg) of chitosan to 0.75 to 1.25 (in ml) of acetic acid diluted to 0.4 to 0.6% by volume in water;
adding the viscous chitosan to the mixture of powders in a proportion by weight of 3 to 5 of powder to 5 to 10 of chitosan;
carrying out a first compression followed by light grinding;
carrying out a second compression to produce a pellet, the pressure applied during the first and the second compression being within a range from 2,000 to 6,000 kPa; and
drying at ambient temperature.

2. The method according to claim 1, wherein the solution comprises from 0.002 to 0.005% in mass per volume of genipin.

3. The method according to claim 1, wherein the solution comprises from 0.001 to 0.005% in mass per volume of caffeic acid.

4. Bioreactor obtained by implementing the method according to any one of claims 1 to 3.

5. Bioreactor according to claim 4, wherein a porous chitosan-based membrane (26) is laid on an active surface of the bioreactor and bonded at the periphery thereof.

6. The bioreactor according to claim 5, forming a pellet-shaped bioelectrode (20), wherein a conductor (22) is glued by means of conductive glue (24) to the surface of the pellet that is opposite to the active surface.

7. The bioreactor according to claim 5 or 6, wherein the membrane comprises pores having an average diameter in the range from 1 to 10 nanometers.

8. The bioreactor according to any one of claims 5 to 7, wherein the membrane comprises a smooth surface facing the pellet and a rough surface facing outwards.

9. The bioreactor according to any one of claims 5 to 8, wherein the porous membrane is obtained by a method comprising the steps of:
preparing a solution in a ratio of 5 to 15 (in mg) of chitosan to 0.75 to 1.25 (in ml) of acetic acid diluted to 0.4 to 0.6% in water;
stirring;
pouring on a smooth support; and
drying for a period from 2 to 4 days at ambient temperature.
